# EUROPEAN PATENT APPLICATION

(11) **EP 4 745 229 A1**
(43) Date of publication of application: **20.05.2026**
(21) Application number: 24839962.8
(22) Date of filing: 01.07.2024
(51) Int. Cl.: C12N 5/077, G01N 33/50

(54) **METHOD FOR PREPARING CARDIAC ORGANOIDS DERIVED FROM HUMAN PLURIPOTENT STEM CELLS, AND EFFICACY AND TOXICITY EVALUATION TECHNIQUE**

(30) Priority: 12.07.2023 KR 20230090709; 27.09.2023 KR 20230130635
(71) Applicant: Biosolvix.,Co.,Ltd, Seoul 08502 (KR)
(72) Inventor: MOON, Sung-hwan, Guri-si, Gyeonggi-do 11940 (KR); LEE, Song Ee, Seoul 08355 (KR)
(74) Representative: Potter Clarkson
(86) International application number: PCT/KR2024/009207
(87) International publication number: WO 2025/014149

(57) **Abstract**

The present disclosure relates to a method for preparing cardiac organoids derived from human pluripotent stem cells, and an efficacy and toxicity evaluation technique. When the cardiomyocytes and cardiac organoids purified by a CD47 surface marker, of the present disclosure, are used, mature cardiomyocytes and cardiac organoids can be formed, and the cardiomyocytes and cardiac organoids are characterized in that mature cardiomyocytes having uniform contractile ability and a large surface area are formed at a high rate, and thus the present disclosure can be effectively used in cardiovascular disease modeling including that in toxicity evaluation and drug screening.

## Description

### [Technical Field]

The present patent application claims priority to Korean Patent Application No. 10-2023-0090709 as filed in the Korean Intellectual Property Office on July 12, 2023, and Korean Patent Application No. 10-2023-0130635 as filed in the Korean Intellectual Property Office on September 27, 2023, the contents of which in their entirety are herein incorporated by reference.

The present disclosure relates to a method for preparing cardiac organoids derived from human pluripotent stem cells, and a technology for evaluating efficacy and toxicity.

### [Background Art]

Stem cells are undifferentiated cells that differentiate into cells having specific functions through cell division or have self-reproducibility (self-renewal) that can be regenerated into cells having undifferentiated and pluripotent functions through cell division. Among the stem cells, human pluripotent stem cells are cells in a stage before they are differentiated into various organs as early fertilized egg cells are divided, and have characteristics that can be divided into all cells of the fetus or adult, and embryonic stem cells and induced pluripotent stem cells (iPSCs) correspond thereto.

Recently, a technology for making organoids, an organ-specific cell aggregate in the body, using pluripotent stem cells has attracted attention. Organoids are organ analogs formed by agglomerating and recombining cells isolated from stem cells or organ-derived cells by a 3D culture method, and are characterized by forming specific cells of organs such as the heart, stomach, intestine, liver, and the like, reproducing specific functions of organs, or enabling spatial organization with a structure similar to that of real organs. Since organoids can mimic organs of a more complex living body than general cultured cells based on their self-organization ability, they are in the spotlight to be used as a good three-dimensional model for understanding the development of organs in the early stages of differentiation, tissue interactions, and disease occurrence.

In particular, it is expected that clinical limitations using existing animal models can be overcome by utilizing similarities with actual organs of organoid. Through a model that reproduces the actual organs of organoid, it is possible to make up for the difficulty of application due to species differences from humans, to solve ethical problems for animal experiments, and to obtain results more suitable for living organisms through organoid with organ-level three-dimensional structures compared to conventional research utilizing monolayer-cultured or simply organized cells.

In addition, since organoids can construct only necessary organs, they are more experimentally accessible than existing bio-based research, and thus it is easy to identify molecular-level mechanisms or to apply the latest research techniques. Due to these characteristics, organoid is an optimal experimental body for identifying the efficacy and stability of a drug in the process of developing a new drug, and can be said to be a next-generation core bio-technology that can be used not only for drug toxicity evaluation and efficacy evaluation in the development of a new drug but also for disease research, cancer treatment, and regenerative medicine.

In the organs reprepared through organoid, organs such as the stomach, intestine, liver, lungs, and brain were currently reprepared. However, in the case of the organoid repreparing the heart, the electrical and physiological characteristics of the heart may not be fully reprepared, the characteristics of immature cardiomyocytes may be exhibited, the formation and arrangement of the myocardium may be structurally incomplete, and the organoid may not be similar to the heart having the atrium and ventricular structure of the living body. In addition, there are disadvantages in that long-term cultivation through a culture medium in which oxygen and nutrients are distributed for forming a vascular network is difficult, the formation of an ion channel is weak, and the reactivity to a drug is incomplete. Moreover, in the case of cardiac organoids prepared using the purification of general cardiomyocytes, it is difficult to derive an accurate result in evaluating toxicity of drugs due to the characteristic that the beating range of general cardiomyocytes is widely exhibited in the beating ability of cardiomyocytes.

Since most cardiovascular diseases are characterized by developing after adulthood, the process of acquiring, culturing, and maturing a sufficient amount of cardiomyocytes with a constant range of beating is required to produce a cardiac organoid for drug and toxicity evaluation of cardiovascular diseases.

Therefore, there is a need to develop a cardiac organoid prepared by inducing differentiation and separation of cardiomyocytes having a constant beating range from pluripotent stem cells with high efficiency and inducing differentiation of the cardiomyocytes.

Against this background, the present inventors discovered that the interval of beating of cardiomyocytes purified with the CD47 surface marker was constant, and thus, the cardiomyocytes were purified with high yield and high efficiency through the cardiomyocyte culture, and completed the present disclosure by preparing cardiac organoid using the same.

### [Disclosure]

### [Technical Problem]

A purpose of the present disclosure is to provide a method for preparing cardiomyocytes purified with the CD47 surface markers and a method for preparing cardiac organoids through the culture process of cardiomyocytes.

Another purpose of the present disclosure is to provide a human pluripotent stem cell-derived cardiac organoid prepared by the preparation method.

Still another purpose of the present disclosure is to provide a method for evaluating the toxicity of a drug using the cardiac organoid.

Still yet another purpose of the present disclosure is to provide a method for screening a candidate drug for treating a heart disease using the cardiac organoid.

### [Technical Solution]

In order to achieve the above purpose, the present disclosure provides a method for preparing cardiomyocytes, the method including steps: i) culturing human pluripotent stem cells to be differentiated into an embryoid body;
ii) culturing the differentiated embryoid body to differentiate into a mesoderm;
iii) culturing the differentiated mesoderm into cardiomyocytes; and
iv) purifying the cultured cardiomyocytes with a CD47 surface marker.

The present disclosure also provides a method for preparing a human pluripotent stem cell-derived cardiac organoid, the method including culturing the cardiomyocytes prepared by the preparation method of the cardiomyocytes in a differentiation medium used for cardiac organoid formation.

The present disclosure also provides a human pluripotent stem cell-derived cardiac organoid prepared through the preparation method.

The present disclosure also provides a method for evaluating toxicity of a drug, the method including: treating the cardiac organoid with a drug; and measuring activity of the organoid.

The present disclosure also provides a method for screening a candidate drug for treating a heart disease, the method including: treating the cardiac organoid with the candidate drug for treating the heart disease; and, by determining whether expression of a biomarker protein of the heart disease or an mRNA encoding the biomarker protein from the human pluripotent stem cell-derived cardiac organoid treated with the candidate drug is increa.sed or decreased compared to before the treatment and upon determination that the expression is increased or decreased, selecting the candidate drug as a therapeutic agent for the heart disease.

### [Advantageous Effects]

Using the method for preparing the cardiomyocytes purified with the CD47 surface marker of the present disclosure; the method for preparing the cardiac organoid by culturing the prepared cardiomyocytes; and the cardiac organoid prepared by the preparation method, mature cardiomyocytes and cardiac organoid may be formed, in which the cardiomyocytes and cardiac organoid exhibit a characteristic in which the form of the mature cardiomyocytes having uniform beating ability and a wide surface area is formed at a high percentage, and thus can be usefully utilized in modeling cardiovascular diseases including toxicity evaluation and drug screening.

### [Description of Drawings]

FIG. 1 is a diagram illustrating a cardiomyocyte-specific surface marker among various commercially available surface markers.
FIG. 2 is a diagram showing a differentiation process of cardiomyocytes purified with an iPSC-derived CD47 surface marker of the present disclosure.
FIG. 3 is a diagram showing a beating interval of the cardiomyocytes purified with the CD47 surface marker.
FIG. 4 is a diagram showing the result of maturation of the cardiomyocytes purified with the CD47 surface marker and the result of immunostaining the matured cardiomyocytes.
FIG. 5 is a diagram showing the result of electrophysiological analysis of cardiomyocytes purified with the CD47 surface marker.
FIG. 6 is a diagram showing the result of toxicity analysis of drugs using cardiomyocytes purified with the CD47 surface marker.
FIG. 7 is a diagram showing a process of preparing cardiac organoids using cardiomyocytes purified with the CD47 surface markers and the prepared cardiac organoid.

### [Best Mode]

A method for preparing cardiomyocytes, the method including steps: i) culturing human pluripotent stem cells to be differentiated into an embryoid body;
ii) culturing the differentiated embryoid body to differentiate into a mesoderm;
iii) culturing the differentiated mesoderm into cardiomyocytes; and
iv) purifying the cultured cardiomyocytes with a CD47 surface marker.

Hereinafter, the present disclosure will be described in detail.

### Method for Preparing Cardiomyocytes

In an aspect of the present disclosure, the present disclosure relates to a method for preparing cardiomyocytes, the method including: i) culturing human pluripotent stem cells to be differentiated into an embryoid body;
ii) culturing the differentiated embryoid body to differentiate into a mesoderm;
iii) culturing the differentiated mesoderm into cardiomyocytes; and
iv) purifying the cultured cardiomyocytes with a CD47 surface marker.

The term "human pluripotent stem cell (hPSC)" used herein may refer to a cell having the ability to differentiate into all cells constituting the human body.

The human pluripotent stem cell may be an embryonic stem cell or an induced pluripotent stem cell (iPSC). Specifically, the embryonic stem cells are induced from an inner cell mass of blastocyst before implantation, and the induced cells can be cultured without limitation and can be pluripotency-differentiated. The induced pluripotent stem cell may refer to a pluripotent differentiated cell generated by dedifferentiation from somatic cells of the body, and may be formed by making somatic cells similar to embryonic stem cells through a reprogramming process such as cell fusion, nuclear substitution, and overexpression of a pluripotency regulatory factor.

In an embodiment of the present disclosure, the human pluripotent stem cell may be any one of embryonic stem cells or induced pluripotent stem cells, and for example, the human pluripotent stem cells may be induced pluripotent stem cells.

The term "differentiation" used herein generally refers to a phenomenon in which a relatively simple system is separated into two or more qualitatively different subsystems, and specifically, it may refer to a phenomenon in which structures or functions are specialized while cells are divided and proliferated and grown, that is, a phenomenon in which the shape or function of a living cell, a tissue, etc. is changed to perform a task given to each other.

The term "cardiomyocyte" used herein may include, without limitation, cells of all differentiation stages of myocardial progenitor cells, fetal cardiomyocytes, or adult cardiomyocytes, which have the ability to become functional cardiomyocytes in the future, and may refer to cells that can be identified by a plurality of markers or standards.

For example, the cells having the characteristic of the cardiomyocytes may be identified by the expression of genes of myocardial-specific transcription factors such as CD47, GATA-4, TEF-1, Tbx-5, MEF2, and MLC-2v, or the expression of proteins such as sarcomere, myosin, troponin I, α-actinin, and ANP, which are myocardial-specific markers.

According to a feature of the present disclosure, the cardiomyocytes express a CD47 surface marker. Thus, it is possible to purify the cardiomyocytes with high purity and high efficiency using the CD47 surface marker.

In an embodiment of the present disclosure, the step i) may include: culturing the human pluripotent stem cells in a first differentiation medium containing Y27632 and EGF and then treating the cultured human pluripotent stem cells with EDTA; and culturing the treated human pluripotent stem cells in a second differentiation medium containing Pluronic F-127, N2 supplement and FBS.

A concentration of Y27632 in the first differentiation medium in the step i) may be in a range of 5 to 20 µM/mL, 5 to 15 µM/mL, or 5 to 10 µM/mL, but is not limited thereto.

A concentration of EGF in the first differentiation medium in the step i) may be in a range of 5 to 50 ng/mL, 5 to 45 ng/mL, 5 to 40 ng/mL, 5 to 35 ng/mL, 5 to 30 ng/mL, or 5 to 25 ng/mL, but is not limited thereto.

The step i) may be performed for 1 to 5 days, for example, may be performed for 1 day, 2 days, 3 days, or 4 days.

A concentration of EDTA in the step i) may be in a range of 0.1 to 1.0 mM, 0.1 to 0.8 mM, or 0.1 to 0.6 mM, but is not limited thereto.

A concentration of the Pluronic F-127 in the second differentiation medium in the step i) may be in a range of 0.01 to 0.1 g/L, 0.01 to 0.08 g/L, or 0.01 to 0.06 g/L, but is not limited thereto.

A concentration of the N2 supplement of the second differentiation medium in the step i) may be in a range of 5 to 20%, 5 to 15%, or 5 to 10%, but is not limited thereto.

A concentration of the FBS in the second differentiation medium in the step i) may be in a range of 5 to 20%, 5 to 15%, or 5 to 10%, but is not limited thereto.

In an embodiment of the present disclosure, the differentiation medium of the step ii) may contain Pluronic F-127, BMP4, and FBS.

A concentration of Pluronic F-127 in the differentiation medium of the step ii) may be in a range of 0.01 to 0.1 g/L, 0.01 to 0.08 g/L, or 0.01 to 0.06 g/L, but is not limited thereto.

A concentration of the BMP4 in the differentiation medium in the step ii) may be in a range of 5 to 50 ng/mL, 5 to 45 ng/mL, 5 to 40 ng/mL, 5 to 35 ng/mL, 5 to 30 ng/mL, or 5 to 25 ng/mL, but is not limited thereto.

A concentration of FBS in the differentiation medium in the step ii) may be in a range of 5 to 20%, 5 to 15%, or 5 to 10%, but is not limited thereto.

The step ii) may be performed for 1 day to 8 days, for example, may be performed for 1 day, 2 days, 3 days, 4 days, 5 days, or 6 days.

In an embodiment of the present disclosure, the step iii) may be a step of culturing the mesoderm in a differentiation medium containing Pluronic F-127 and FBS such that the mesoderm is differentiated into the cardiomyocytes.

A concentration of the Pluronic F-127 in the differentiation medium of the step iii) may be in a range of 0.01 to 0.1 g/L, 0.01 to 0.08 g/L, or 0.01 to 0.06 g/L, but is not limited thereto.

A concentration of FBS in the differentiation medium in the step iii) may be in a range of 5 to 20%, 5 to 15%, or 5 to 10%, but is not limited thereto.

In one embodiment of the present disclosure, the differentiated cardiomyocytes may be beating cardiomyocytes.

In an embodiment of the present disclosure, the step iv) may include confirming expression of the CD47 surface marker in the cardiomyocytes differentiated in the step iii); and isolating and purifying the CD47-positive cells, in which the expression of the CD47 surface marker is confirmed, at a cell level.

The term "positive" herein means that a protein or gene is expressed in a detectable amount by at least any method known in the art. Proteins can be detected by immunological assays with antibodies such as ELISA, immunostaining, or flow cytometry. In the case of a protein (e.g., a transcription factor or a subunit thereof) that is expressed in a cell and does not appear on a cell surface, a reporter protein is expressed together with the protein, and a target protein may be detected by detecting the reporter protein. The gene may be detected, for example, by a nucleic acid amplification method such as RT-PCR, a biochip (e.g., a microarray), RNA seq, or the like, and/or a nucleic acid detection method. The expression of a protein or gene may be determined by a general method. For example, if flow cytometry is used and the expression level is relatively high compared to the expression level of a control exhibiting negative protein expression, the protein may be determined to be detectably expressed.

The term "purification" as used herein refers to classifying differentiated cardiomyocytes into CD47- and CD47+ (positive) cardiomyocytes, and selecting only the CD47+ (positive) cardiomyocytes purely from among the CD47- and CD47+ (positive) cardiomyocytes. In an embodiment of the present disclosure, "purification of the cardiomyocytes with the CD47 surface marker" may refer to selecting at least 50%, 55%, 60% or 65%, preferably, at least 70% of the CD47+ (positive) cardiomyocytes from the differentiated cardiomyocytes.

In an embodiment of the present disclosure, the cardiomyocytes purified with the CD47 surface marker of the step iv) may beat 60 to 80 times per minute, for example, 65 to 78 times, but is not limited thereto.

In an embodiment of the present disclosure, the cardiomyocytes purified with the CD47 surface marker of the step iv) may be capable of being used to evaluate the stability against the cardiotoxic drug, but is not limited thereto.

For example, the cardiotoxic drug may be nifedipine, dofetilide, mexiletine, terfenadine, isoproterenol, doxorubicin, daunorubicin, idarubicin, Quinidine, cyclophosphamide, capecitabine, 5-fluorouracil, Quinidine, and Remdesivir, and is not limited thereto. The cardiomyocytes purified with the CD47 surface marker of the step iv) may be used to evaluate the stability against all drugs which may be subjected to the cardiotoxic evaluation.

The term "stability" used herein means that the cardiomyocytes exhibit a constant field potential, a field potential duration, and an Na+ peak (amplitude) value within an error range set for the drug with cardiotoxicity and may be used to evaluate the cardiotoxicity of the drug.

In an embodiment of the present disclosure, the cardiomyocytes purified with the CD47 surface marker in the step iv) may have a mature ventricular-like cardiomyocyte distribution of 75% or greater, but are not limited thereto.

In the method for preparing the cardiomyocytes of the present disclosure, the CD47 may be used as a surface marker specifically expressed in the beating cardiomyocytes. Thus, when the cardiomyocytes are stained with the CD47 marker, cells that do not express the CD47, i.e., CD47-negative cells, may be undifferentiated stem cells or embryoid bodies or non-beating cardiomyocytes. According to the method for preparing the cardiomyocytes of the present disclosure, a case in which the differentiation from the stem cells to cardiomyocytes is not induced and/or a case in which the differentiation from the stem cells to cardiomyocytes occurs but differentiation from the stem cells into the non-beating cardiomyocytes which do not exhibit the beating occurs, may be simply excluded.

### Method for Preparing Cardiac Organoid

In another aspect, the present disclosure relates to a method for preparing a cardiac organoid, the method including a step of culturing the cardiomyocytes prepared by the method for preparing the cardiomyocytes in a differentiation medium used for cardiac organoid formation to form a human pluripotent stem cell-derived cardiac organoid.

The term "organoid" as used herein may refer to a small culture having a structure similar to a biological (human) organ, a composition and a function of a cell, and may have an environment similar to that of an actual biological organ in a laboratory as a three-dimensional structure. The organoid may have an environment in which they are allowed to interact with the surrounding environment in the process of cell growth, and may be preclinical and clinical models capable of observing the development of a therapeutic agent for a disease and the evaluation of toxicity by almost completely simulating an organ interacting *in vivo.*

The method for preparing the cardiac organoid for differentiating the cardiomyocytes into the cardiac organoid may be used without limitation as long as it is a method that can be adopted for preparing the cardiac organoid according to the prior art. Specifically, in order to form the cardiac organoid from the cardiomyocytes, as in the method disclosed in Korean Patent No. 10-2155868 (Patent Document 1), aggregation of vascular endothelial cells may be induced for 24 hours using a microfluidic chip for cell culture, and then the cardiomyocytes purified with the CD47 surface markers may be additionally aggregated for 24 hours to form the cardiac organoid.

In an embodiment of the present disclosure, the differentiation medium used for cardiac organoid formation refers to a cell culture support, and the preparation method may include using a three-dimensional cell culture microfluidic chip including a seeding step of sequentially seeding two or more types of cells into the cell culture support.

In the method of using the 3D cell culture microfluidic chip, the seeding step may specifically include following steps (a) and (b).
(a) aggregation step of seeding and culturing vascular endothelial cells; and
(b) co-culturing step of seeding and culturing the cardiomyocytes.

### Cardiac Organoid Prepared by Preparation Method

In another aspect, the present disclosure relates to a human pluripotent stem cell-derived cardiac organoid prepared by the preparation method.

In an embodiment of the present disclosure, in evaluating the expression amount and functionality of a cardiomyocyte-specific gene, the cardiac organoid formed from the cardiomyocytes expressing the CD47 surface marker may form the cardiomyocytes having a constant beating interval compared to the conventional models used in the drug efficacy and toxicity evaluation, and thus may be more similar in form and function to biological organs.

The cardiac organoid is characterized in that it maintains beats at an interval of 1 second, which is similar to that of the hearts of adults. When the vascular endothelial cells are present inside the organoid, the beats are maintained for at least two months as compared to when only the cardiomyocytes are aggregated to form the cardiac organoid. Thus, drug reactions of various drugs may be analyzed for a long time using the cardiac organoid of the present disclosure.

The cardiac organoid of the present disclosure may have efficacy to be used for evaluating toxicity of a drug in response to the drug; and have efficacy to be used for screening a candidate drug for treating a heart disease, in response to the drug.

The heart disease may be at least one selected from the group consisting of coronary artery disease, hypertension, angina, myocardial infarction, heart valve disease, heart failure, cardiac hypertrophy, arrhythmia, pericarditis, and endocarditis, but is not limited thereto.

### Method for Evaluating Toxicity of Drugs

In another aspect, the present disclosure relates to a method for evaluating toxicity of a drug, the method including: treating the cardiac organoid with the drug; and measuring activity of the organoid.

The term "drug" as used herein may include individual nucleic acids, proteins, other extracts, or natural products that are estimated or randomly selected to have the potential for preventing or treating the heart disease according to a conventional selection method; and commercially available drugs that are estimated or identified to have side effects of toxicity.

The heart disease may be at least one selected from the group consisting of coronary artery disease, hypertension, angina, myocardial infarction, heart valve disease, heart failure, cardiac hypertrophy, arrhythmia, pericarditis, and endocarditis, but is not limited thereto.

### Method for Screening Candidate Drug for Treating Heart Disease

In another aspect, the present disclosure relates to a method for screening a candidate drug for treating a heart disease, the method including: treating the cardiac organoid with the candidate drug for treating the heart disease; and determining whether expression of a biomarker protein of the heart disease or an mRNA encoding the biomarker protein from the human pluripotent stem cell-derived cardiac organoid treated with the candidate drug is increased or decreased compared to before the treatment; and upon determination that the expression is increased or decreased, selecting the candidate drug as a therapeutic agent for the heart disease.

The term "candidate drug" herein may include any substance used to change or review a physiological system or disease state for the benefit of a living organism. For example, it may be a drug candidate substance, a subject compound to be tested, or a subject composition to be tested.

The drug candidate substance may be one or more selected from the group consisting of a cardiac agent, a hyperlipidemia agent, a diuretic, a vitamin, a vaccine, a steroid, an antibiotic, an anticancer agent, an antihistamine, a chemotherapy agent, a blood pressure lowering agent, and a hormone agent, but is not limited thereto.

The subject compound to be tested or the subject composition to be tested may be one or more selected from the group consisting of a low molecular weight compound, an antibody, an antisense nucleotide, a short interfering RNA (siRNA), a short hairpin RNA, hexane, protein, peptide, other extracts, and natural extracts, but is not limited thereto.

The heart disease may be at least one selected from the group consisting of coronary artery disease, hypertension, angina, myocardial infarction, heart valve disease, heart failure, cardiac hypertrophy, arrhythmia, pericarditis, and endocarditis, but is not limited thereto.

Hereinafter, the present disclosure will be described in more detail through Examples. These Examples are only for illustrating the present disclosure, and the contents of the present disclosure are not limited by these Examples.

### Example 1. Discovery of Cardiomyocyte-Specific Surface Marker

Differentiation from iPSC into cardiomyocytes was induced, and then each cell was subjected to lysis to obtain a cell extract, and a global transcriptome assay was performed thereon to determine the expression level of mRNA contained therein. From the result of the global transcriptome assay, the result of the cardiac and heart-related gene clusters of the global surface marker (CD marker) gene expression profile were derived using the mean of the genes and the deviation of the genes.

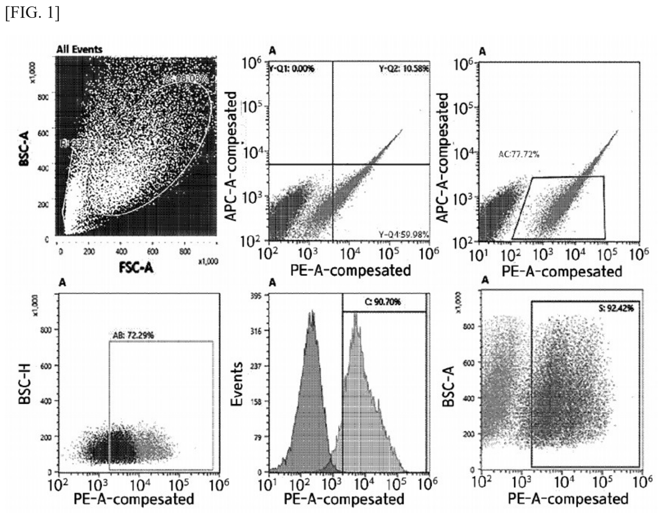

As a result of the marker analysis, it was identified that the CD47 surface marker was specifically expressed in most of the cardiomyocytes (FIG. 1). Therefore, in order to prepare the cardiomyocytes purified with the CD47 surface marker, the following examples were performed.

### Example 2. Induction of Differentiation from Stem Cells into Cardiomyocytes

### 2-1. Embryoid Body Formation

First, in order to form the human embryoid body (hEB) for cardiomyocyte differentiation, undifferentiated iPSCs (Wicell, KDCA; KNIH) were cultured in a medium containing 10 µM/mL Y27632 (Tocris) and 10 ng/mL EGF (R&D systems). After 1 day, treatment with 0.5 mM EDTA (Gibco) was performed thereon, followed by culturing thereof in a culture solution containing 10% N2 supplement (Gibco) and 8% FBS (Hyclone) in a 0.05 g/L Pluronic F-127 (Sigma)-coated culture dish to obtain the resultant embryoid body.

### 2-2. Induction of Differentiation into Mesodermal and Cardiomyocyte

After identifying the formation of the embryoid body, the 0.05 g/L Pluronic F-127-coated culture dish was replaced on day 4, and then the embryoid body was cultured in a culture medium containing 12 ng/mL BMP4 (R&D systems) and 8% FBS (Hyclone) such that the mesoderm was induced. Thereafter, for cardiomyocyte enhancement, the Pluronic F-127-coated culture dish was replaced on day 6, and then the mesoderm was cultured with culture medium containing only FBS, and the same procedure was performed on days 8, 10, and 12 to obtain differentiated cardiomyocytes.

### 2-3. Beating Cardiomyocyte Purification

The obtained cardiomyocytes started to beat from day 10, and after identifying that the beating occurred, the cardiomyocytes were dissociated with TrypLE (Gibco) and washed with PBS. The dissociated cells were suspended in PBS containing 5% fetal bovine serum (FBS, High Clone), and the cardiomyocytes were isolated using the cardiomyocyte-specific surface marker CD47 identified in Example 1 above using the SH800S cell sorter flow cytometer and cell sorter software Ver 2.1.2 (Sony Biotechnology). By flow cytometry, approximately 70% or greater of the cells were observed as the CD47+ cells.

### Example 3. Analysis of Cardiomyocyte Purified with CD47 Surface Marker

### 3-1. Analysis of Beating Interval and Form

In order to analyze the beating interval of cardiomyocytes purified with the CD47 surface marker, the beating of the cardiomyocytes observed for 1 minute was quantified using a real-time microscope, and the results were shown in Table 1 below.

**[Table 1]**

| Classification | Interval (sac) | | Contracting No. (1 min) | |
|---|---|---|---|---|
| | Purification with culture medium | Purification with CD47 | Purification with culture medium | Purification with CD47 |
| No.1 | 0.68 | 0.86 | 88 | 69 |
| No.2 | 0.76 | 0.88 | 78 | 68 |
| No.3 | 1.02 | 0.90 | 58 | 66 |
| No.4 | 0.88 | 0.82 | 68 | 73 |
| No.5 | 0.80 | 0.78 | 75 | 76 |
| Range (Interval) | 0.68 to 1.02 (0.34) | 0.78 to 0.90 (0.12) | 58 ~ 88 (30) | 66 ~ 76 (10) |

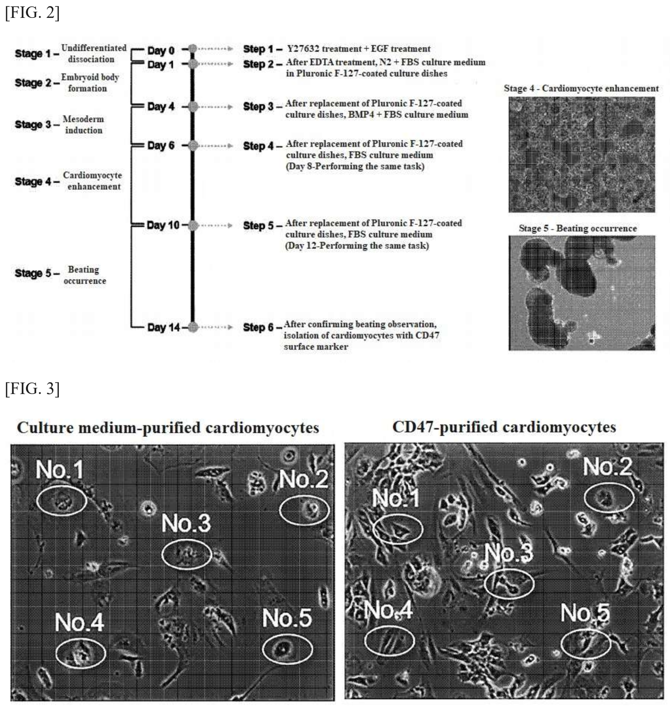

As shown in Table 1 and FIG. 3 of the present disclosure, CD47-purified cardiomyocytes exhibited 66 to 76 beatings per minute, and the beating interval was 10 times, which was close to about 60 to 80 times, which is a heart rate per minute of a general adult, but the general culture medium-purified cardiomyocytes exhibited 58 to 88 beatings per minute and the beating interval was 30 times, which was different from the adult heart rate. Therefore, it was identified that the beating interval of the cardiomyocytes purified with the CD47 surface marker was constant as that of the actual heart is, compared to the cardiomyocytes purified with the general cardiomyocyte culture medium.

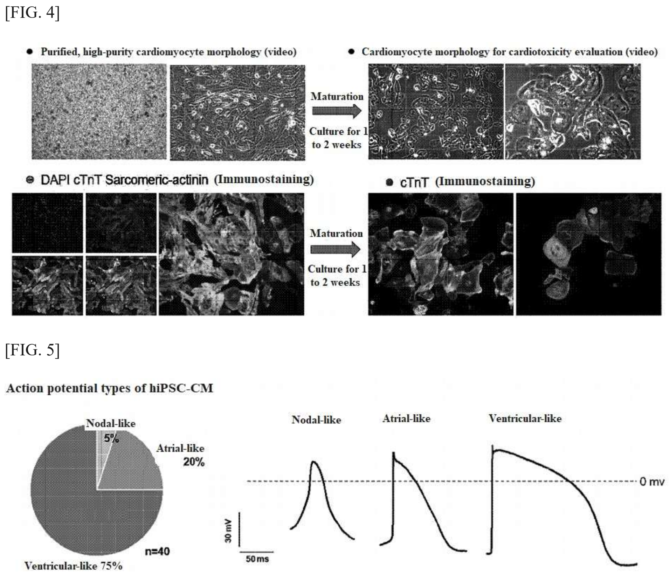

In addition, as a result of the culture of the CD47-purified cardiomyocytes with 10% N2 supplement (Gibco) and 3% FBS (Hyclone) for 2 weeks, it was identified, as shown in FIG. 4 of the present disclosure, that 90% or greater of cells of the CD47-purified cardiomyocytes were observed as the mature cardiomyocytes only with 2 weeks of the culture.

### 3-2. Electrophysiological Analysis

Electrophysiological analysis was performed on the mature cardiomyocytes analyzed in Example 3-1.

Specifically, the cardiomyocytes classified using the CD47 surface marker were analyzed by soaking the cardiomyocytes in pH 7.4 mOsm containing 130 mM NaCl, 5 mM KCl, 1 mM MgCl₂, 1.8 mM CaCl₂, 10 mM HEPES, and 12.5 mM glucose as the external solution under 37°C condition. To create a cell patch clamp condition, a micro glass tube adhering to the cell membrane was filled with an internal pipette solution composed of 120 mM K-aspartate, 20 mM KCl, 10 mM HEPES, 5 mM EGTA, 1 mM MgCl₂, 1.5 mM Mg-ATP, pH 7.3. Then, the voltage and current of the cardiomyocyte were recorded using the Axopatch 200B amplifier, Digidata 1440A, and pClamp software 10.1 (Axon Instruments, USA), and the current was measured in the order of the resting membrane potential of -80 mV, depolarization at +40 mV for 4 seconds, and repolarization at -40 mV for 2 seconds, and the results were shown in Table 2 and FIG. 5 below.

**[Table 2]**

| | Amplitude (mV) | APD 80 (ms) | Vmax (mV/ms) | MDP (mV) | Beat period (ms) |
|---|---|---|---|---|---|
| Atriallike hiPSC-CMs | 85.21 ± 7.25 | 131.66 ± 18.41 | 28.95 ± 6.20 | -58.11 ± 3.46 | 234.08 ± 65.82 |
| Ventricular likehiPSC-CMs | 109.13 ± 9.43 | 220.43 ± 15.60 | 43.27 ± 9.32 | -69.10 ± 5.61 | 648.97 ± 81.20 |

As shown in Table 2 and FIG. 5 of the present disclosure, the cardiomyocytes purified with the CD47 surface marker exhibited electrical signals of all three types of cardiomyocytes, and it was observed that the ventricular-like cardiomyocytes among the three types of cell groups were dominant at a high ratio of 75% or greater, such that the cardiomyocytes purified with the CD47 surface marker had a function of the mature ventricular cardiomyocytes.

### Example 4. Toxicity Analysis using Cardiomyocytes Purified with CD47 Surface Marker

The CD47-purified cardiomyocytes were used to analyze the cardiotoxicity of the drug including Remdesivir (MedChem Express) and Quinidine (Sigma-Aldrich) known as cardiotoxic drugs.

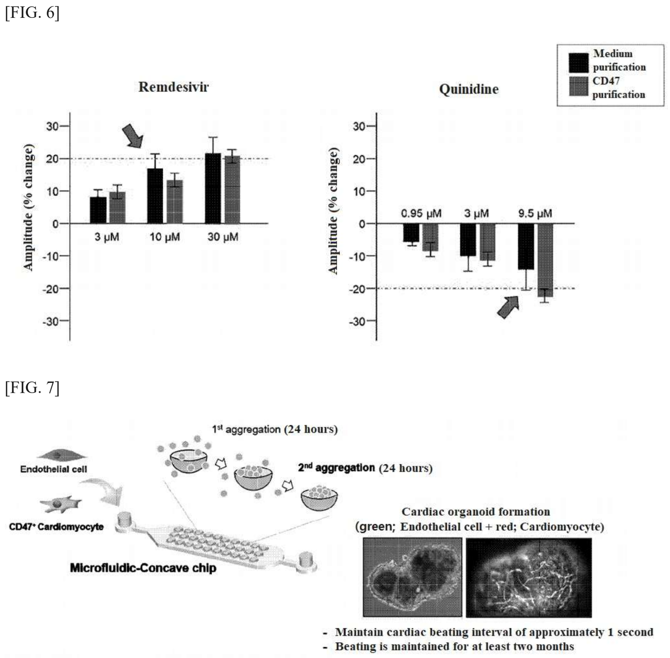

A field potential (FP) measured as a spontaneous action potential of the CD47+ cardiomyocytes was recorded through a multi-electrode array (Maestro Pro, Axion BioSystem; MEA). The FP signal was analyzed using the cardiac analysis tools 2.2.7 program (Axis Biosystems), and the analysis was expressed as a percent change in the analysis values of FP duration (FPD), modified FPD (FPDc; formula of Fredericia), beat period, and Na+ peak (amplitude) according to the treatment of the cardiomyocytes with each of the concentrations (3 µM, 10 µM, and 30 µM) of Remdesivir drug and the treatment of the cardiomyocytes with each of the concentrations (0.95 µM, 3 µM, and 9.5 µM) of Quinidine drug in FIG. 6, and the results were shown in Table 3, Table 4 below and FIG. 6 of the present disclosure.

**[Table 3]**

| Amplitude (% change) | Remdesivir | |
|---|---|---|
| | Purification with culture medium | Purification with CD47 |
| 3 µM | 8 ± 2 | 9 ± 1.5 |
| 10 µM | 17 ± 3.5 | 12 ± 1.5 |
| 30 µM | 21 ± 3 | 20 ± 1.5 |

**[Table 4]**

| Amplitude (% change) | Quinidine | |
|---|---|---|
| | Purification with culture medium | Purification with CD47 |
| 0.95 µM | -5 ± 1 | -8 ± 1.5 |
| 3 µM | -10 ± 3.5 | -12 ± 1.5 |
| 9.5 µM | - 15 ± 5.5 | -22 ± 1.5 |

In the case of Remdesivir, the risk of cardiotoxicity was detected in the error range at a concentration of 10 µM, but the cardiotoxicity according to the error range was not detected in the CD47-purified cardiomyocytes, and in the case of Quinidine, the risk of cardiotoxicity was detected at a concentration of 9.5 µM in the CD47 cardiomyocytes, but in the case of general culture medium-based purification, the risk of cardiotoxicity was detected in the error range, so there might be limitations in securing reliability and stability in the toxicity result using cardiomyocytes in the general culture medium-based purification, but in the case of CD47 cardiomyocytes, the cardiotoxicity was detected in a stable range such that the efficacy of evaluating the toxicity of the drug using the CD47 cardiomyocytes could be identified.

### Example 5. Preparation of Cardiac Organoid using Cardiomyocytes Purified with CD47 Surface Marker

In order to produce the cardiac organoid using cardiomyocytes purified with the CD47 surface marker, the three-dimensional cell culture microfluidic chip was used.

First, the vascular endothelial cells were seeded into the microfluidic chip for cell culture and were aggregated, and on the second day, CD47+ cardiomyocytes purified with the CD47 surface marker were co-cultured therewith (FIG. 7).

Specifically, the vascular endothelial cells were treated with 0.25% trypsin and incubated at 37°C for 2 mins, centrifuged at 1,000 rpm for 5 min, and resuspended in medium to a final concentration of 5 × 10⁵ cells/mL. Thereafter, two days later, 200 mL of the differentiated CD47+ cardiomyocytes in Example 2 were seeded and cultured at 37°C for one day, and then were additionally seeded into a culture support containing the vascular endothelial cells, followed by the co-culturing. The cell culture fluid was injected through the inlet of the microfluidic chip at a rate of 0.8 µL/min, and 500 to 1,000 µL of fresh culture fluid was added to the culture fluid storage container placed at the inlet every 12 hours.

When the aggregation is induced for 24 hours after the co-culture, the cardiac organoid in which the vascular cells exist in the inner area and the cardiomyocytes exist in the outer area is formed, and the form thereof is shown in FIG. 7 of the present disclosure.

It may be identified that the cardiomyocytes purified with the CD47 surface marker are located in the outer area of the formed cardiac organoid, such that the cardiac organoid may maintain a regular beat of about 1 second, in a similar manner to the heart of an adult, and the beat is maintained for two months or more due to the presence of the vascular endothelial cells present in the inner area of the formed cardiac organoid. Thus, the cardiac organoid including the cardiomyocytes purified with the CD47 surface marker may be used for long-term analysis in the various drug screening and the cardiotoxicity evaluation of the drugs.

Although the present disclosure has been described in detail through the embodiments above, those skilled in the art will understand that various modifications are possible to the above-described embodiments within the scope of the present disclosure. Therefore, the scope of the present disclosure should not be limited to the above-described embodiments, but should be determined by all changes or modifications derived from the claims and the concept of equality as well as the claims to be described later.

### [Industrial Availability]

A purpose of the present disclosure is to provide a method for preparing cardiomyocytes purified with the CD47 surface markers, and a method for preparing cardiac organoid through the culture process of the cardiomyocytes.

Another purpose of the present disclosure is to provide a human pluripotent stem cell-derived cardiac organoid prepared by the preparation method.

Still another purpose of the present disclosure is to provide a method for evaluating the toxicity of the drug using the cardiac organoid.

Still yet another purpose of the present disclosure is to provide a method for screening a candidate drug for treating a heart disease using the cardiac organoid.

## Claims

1. A method for preparing cardiomyocytes, the method comprising steps:
i) culturing human pluripotent stem cells to be differentiated into an embryoid body;
ii) culturing the differentiated embryoid body to differentiate into a mesoderm;
iii) culturing the differentiated mesoderm into the cardiomyocytes; and
iv) purifying the cultured cardiomyocytes with a CD47 surface marker.

2. The method for preparing cardiomyocytes of claim 1, wherein the human pluripotent stem cell is any one of embryonic stem cells or induced pluripotent stem cells.

3. The method for preparing cardiomyocytes of claim 1, wherein the step i) includes:
culturing the human pluripotent stem cells in a first differentiation medium containing Y27632 and EGF and then treating the cultured human pluripotent stem cells with EDTA; and
culturing the treated human pluripotent stem cells in a second differentiation medium containing Pluronic F-127, N2 supplement and FBS.

4. The method for preparing cardiomyocytes of claim 1, wherein a differentiation medium used in the step ii) contains Pluronic F-127, BMP4, and FBS.

5. The method for preparing cardiomyocytes of claim 1, wherein the step iii) is a step of culturing the mesoderm in a differentiation medium containing Pluronic F-127 and FBS such that the mesoderm is differentiated into the cardiomyocytes.

6. The method for preparing cardiomyocytes of claim 5, wherein the differentiated cardiomyocytes are beating cardiomyocytes.

7. The method for preparing cardiomyocytes of claim 1, wherein the step iv) includes:
confirming expression of the CD47 surface marker in the cardiomyocytes differentiated in the step iii); and
isolating and purifying CD47-positive cells, in which the expression of the CD47 surface marker is confirmed, at a cell level.

8. The method for preparing cardiomyocytes of claim 1, wherein the cardiomyocytes purified with the CD47 surface marker in the step iv) beat 60 to 80 times per minute.

9. The method for preparing cardiomyocytes of claim 1, wherein the cardiomyocytes purified with the CD47 surface marker in the step iv) may be used to evaluate stability against a cardiotoxic drug.

10. The method for preparing cardiomyocytes of claim 1, wherein the cardiomyocytes purified with the CD47 surface marker in the step iv) have a mature ventricular-like cardiomyocyte distribution of 75% or greater.

11. A method for preparing a cardiac organoid, the method comprising culturing the cardiomyocytes prepared by the preparation method of claim 1 in a differentiation medium used for cardiac organoid formation to form a human pluripotent stem cell-derived cardiac organoid.

12. A human pluripotent stem cell-derived cardiac organoid prepared by the preparation method of claim 11.

13. The human pluripotent stem cell-derived cardiac organoid of claim 12, wherein the cardiac organoid has efficacy to be used for evaluating toxicity of a drug in response to the drug; and has efficacy to be used for screening a candidate drug for treating a heart disease, in response to the drug.

14. The human pluripotent stem cell-derived cardiac organoid of claim 13, wherein the heart disease is at least one selected from the group consisting of coronary artery disease, angina, myocardial infarction, heart valve disease, heart failure, cardiac hypertrophy, arrhythmia, pericarditis, and endocarditis.

15. A method for evaluating toxicity of a drug, the method comprising:
treating the human pluripotent stem cell-derived cardiac organoid according to claim 12 with the drug; and
measuring activity of the human pluripotent stem cell-derived cardiac organoid.

16. A method for screening a candidate drug for treating a heart disease, the method comprising:
the human pluripotent stem cell-derived cardiac organoid according to claim 12 with the candidate drug for treating the heart disease; and
determining whether expression of a biomarker protein of the heart disease or an mRNA encoding the biomarker protein from the human pluripotent stem cell-derived cardiac organoid treated with the candidate drug is increased or decreased compared to before the treatment and upon determination that the expression is increased or decreased, selecting the candidate drug as a therapeutic agent for the heart disease.
